(19) Europäisches Patentamt
European Patent Office
Office européen des brevets

(11) **EP 3 176 553 B1**

## (12) EUROPÄISCHE PATENTSCHRIFT

(45) Veröffentlichungstag und Bekanntmachung des Hinweises auf die Patenterteilung:
**08.08.2018 Patentblatt 2018/32**

(51) Int Cl.:
***G01K 1/14*** *(2006.01)*

(21) Anmeldenummer: **15197472.2**

(22) Anmeldetag: **02.12.2015**

(54) **SENSOR-ANORDNUNG**

SENSOR ASSEMBLY

SYSTEME DE CAPTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**07.06.2017 Patentblatt 2017/23**

(73) Patentinhaber: **E+E Elektronik Ges.m.b.H.**
**4209 Engerwitzdorf (AT)**

(72) Erfinder: **HAIDER, Albin**
**4211 Alberndorf (AT)**

(74) Vertreter: **Hofmann, Ernst**
**Dr. Johannes Heidenhain GmbH,**
**Patentabteilung,**
**Postfach 12 60**
**83292 Traunreut (DE)**

(56) Entgegenhaltungen:
**EP-A1- 2 657 691      EP-A1- 2 801 804**
**WO-A2-2004/090679**

**Beschreibung**

## GEBIET DER TECHNIK

Die vorliegende Erfindung betrifft eine Sensor-Anordnung, die insbesondere zur Erfassung von Klimaparametern geeignet ist.

## STAND DER TECHNIK

Aus der EP 2 657 691 A1 ist eine Sensor-Anordnung bekannt, die einen kapazitiven Feuchtesensor umfasst, der auf einem integrierten Signalverarbeitungsbaustein angeordnet ist. Der Signalverarbeitungsbaustein weist desweiteren einen darin integrierten Temperatursensor auf. Um eine derartige Sensor-Anordnung gegen Beschädigung zu schützen und eine automatisierte Montage derselbigen z.B. auf einer Leiterplatte zu ermöglichen, wird der Signalverarbeitungsbaustein üblicherweise auf einem Trägerelement (Die-Pad) eines Systemträgers (Leadframe) angeordnet und mit einer geeigneten Umkapselung (Mold) versehen. Zum Systemträger gehören neben dem Trägerelement dabei auch noch Anschlusskontakte (Leads) zur elektrischen Kontaktierung des Signalverarbeitungsbausteins. In Bezug auf derartige Anordnungen, auch Packages genannt, sei beispielsweise auf die DE 20 2014 102 842 U1 verwiesen.

## ZUSAMMENFASSUNG DER ERFINDUNG

Aufgabe der vorliegenden Erfindung ist es, eine Sensor-Anordnung zur Erfassung von Klimaparametern dahingehend weiterzubilden, dass deren Einsatzmöglichkeiten erweitert werden.

Diese Aufgabe wird durch eine Sensor-Anordnung mit den Merkmalen des Anspruchs 1 gelöst.

Vorteilhafte Ausführungsformen der erfindungsgemäßen Sensor-Anordnung ergeben sich aus den Maßnahmen, die in den von Anspruch 1 abhängigen Ansprüchen aufgeführt sind.

Die erfindungsgemäße Sensor-Anordnung weist einen Systemträger auf, der mindestens ein erstes planares Trägerelement und ein zweites planares Trägerelement umfasst, wobei die Trägerelemente in einer Anordnungsebene beabstandet voneinander angeordnet sind. Ferner ist ein integrierter Signalverarbeitungsbaustein vorgesehen, der auf den Trägerelementen angeordnet ist sowie eine Umkapselung, die den Signalverarbeitungsbaustein mindestens teilweise umschließt. Im Signalverarbeitungsbaustein sind mindestens zwei Temperatursensoren integriert, wobei je ein Temperatursensor benachbart zu einem Trägerelement angeordnet ist.

Es kann vorgesehen werden, dass gegenüberliegende Randbereiche des Signalverarbeitungsbausteins auf den Trägerelementen aufliegen.

Hierbei kann in den Auflagebereichen zwischen dem Signalverarbeitungsbaustein und den Trägerelementen ein Befestigungsmaterial angeordnet sein, welches einen geringen thermischen Widerstand besitzt.

Es ist ferner möglich, dass der Signalverarbeitungsbaustein einen rechteckförmigen Querschnitt aufweist und diejenigen Randbereiche des Signalverarbeitungsbausteins auf den Trägerelementen aufliegen, die sich entlang der Rechtecks-Längsachse gegenüberliegen.

Dabei können die Trägerelemente jeweils einen rechteckförmigen Querschnitt aufweisen und die Längsachsen der Trägerelemente rechtwinklig zur Längsachse des Signalverarbeitungsbausteins angeordnet sein.

Desweiteren kann die Länge der Trägerelemente entlang ihrer Längsachse jeweils größer als die Breite des Signalverarbeitungsbausteins senkrecht zur Rechtecks-Längsachse sein.

Es ist ferner möglich, dass die Trägerelemente in der Anordnungsebene einen gegenseitigen Abstand zueinander aufweisen, der derart gewählt ist, dass eine thermische Entkopplung zwischen den Trägerelementen gewährleistet ist.

Desweiteren erweist sich als günstig, wenn zwischen den Trägerelementen ein Material angeordnet ist, das eine geringe Wärmeleitfähigkeit aufweist.

Dabei ist es möglich, dass die Trägerelemente aus einer Kupfer-Legierung oder einer Nickel-Eisen-Legierung bestehen.

Es kann auch vorgesehen werden, dass der Bereich zwischen den Trägerelementen baulich derart ausgebildet ist, dass dort eine verringerte Wärmeleitfähigkeit gegenüber den Trägerelementen resultiert.

In einer vorteilhaften Ausführungsform ist auf dem Signalverarbeitungsbaustein ein gassensitiver Sensor angeordnet und mit diesem elektrisch leitfähig verbunden.

Dabei kann die Umkapselung quaderförmig ausgebildet sein und den Systemträger mit den Trägerelementen und dem Signalverarbeitungsbaustein mit Ausnahme einer Kavität formschlüssig umgeben, wobei sich die Kavität vom Sensor bis zu einer begrenzenden Seitenfläche der Umkapselung erstreckt.

Es ist ferner möglich, ein System auszubilden, bestehend aus einer Sensor-Anordnung wie vorstehend charakterisiert, angeordnet auf einem plattenförmigen Substrat, wobei das Substrat im Bereich eines Trägerelements Wärmeleitungsmittel aufweist, über die ein verbesserter Wärmeübergang zwischen dem Substrat und der Umgebung erzielbar ist.

Es ist hierbei möglich, dass die Wärmeleitungsmittel mindestens eine zusätzliche Metallschicht im Substrat umfassen.

Desweiteren können die Wärmeleitungsmittel mehrere Durchkontaktierungen im Substrat umfassen, die zumindest teilweise mit einem Material hoher Wärmeleitfähigkeit gefüllt sind.

Als besonders vorteilhaft an der erfindungsgemäßen Sensor-Anordnung erweist sich, dass darüber erweiterte Einsatzmöglichkeiten geschaffen werden. So ist es nunmehr möglich, vielfältige Temperaturmessungen über die mindestens zwei im Signalverarbeitungsbaustein integrierten Temperatursensoren vorzunehmen. Beispielsweise können etwa zwei unterschiedliche Temperaturen außerhalb des Signalverarbeitungsbausteins gemessen werden. Ferner ist es möglich, die Eigenerwärmung des Signalverarbeitungsbausteins zu erfassen. Desweiteren kann der Einfluss externer Wärmequellen auf die Messung der Klimaparameter kompensiert werden. Schließlich ist es möglich, durch eine im Signalverarbeitungsbaustein integrierte Heizung und über die Messung von mindestens zwei Temperaturen auf die Strömungsgeschwindigkeit der die Sensor-Anordnung umgebenden Luft zu schließen etc..

Weitere Einzelheiten und Vorteile der vorliegenden Erfindung seien anhand der nachfolgenden Beschreibung von Ausführungsbeispielen der erfindungsgemäßen Sensor-Anordnung in Verbindung mit den Figuren erläutert.

## KURZE BESCHREIBUNG DER ZEICHNUNGEN

Es zeigt hierbei

Figur 1a, 1b      je eine perspektivische Ansicht eines Ausführungsbeispiels der erfindungsgemäßen Sensor-Anordnung;

Figur 2      eine Draufsicht auf die Anordnungsebene der Trägerelemente der Sensor-Anordnung aus den Figuren 1a, 1b;

Figur 3      eine seitliche Schnittansicht der Sensor-Anordnung aus den Figuren 1a, 1b;

Figur 4a      das thermische Ersatzschaltbild der Sensor-Anordnung aus den Figuren 1a, 1b;

Figur 4b      ein vereinfachtes thermisches Ersatzschaltbild der Sensor-Anordnung aus den Figuren 1a, 1b;

Figur 5      ein System mit einer Sensor-Anordnung gemäß dem ersten Ausführungsbeispiel;

Figur 6a      das thermische Ersatzschaltbild des Systems aus Fig. 5;

Figur 6b      ein vereinfachtes thermisches Ersatzschaltbild des Systems aus Fig. 5.

## BESCHREIBUNG DER AUSFÜHRUNGSFORMEN

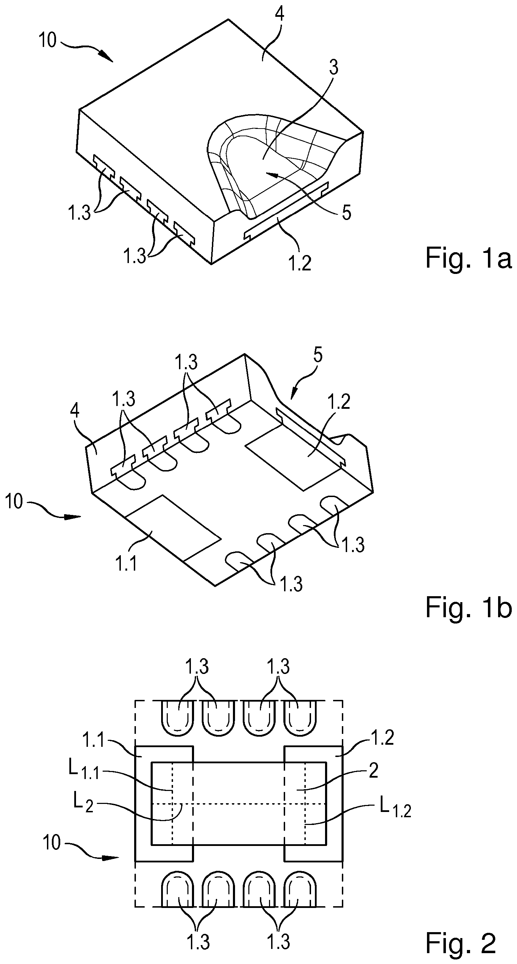

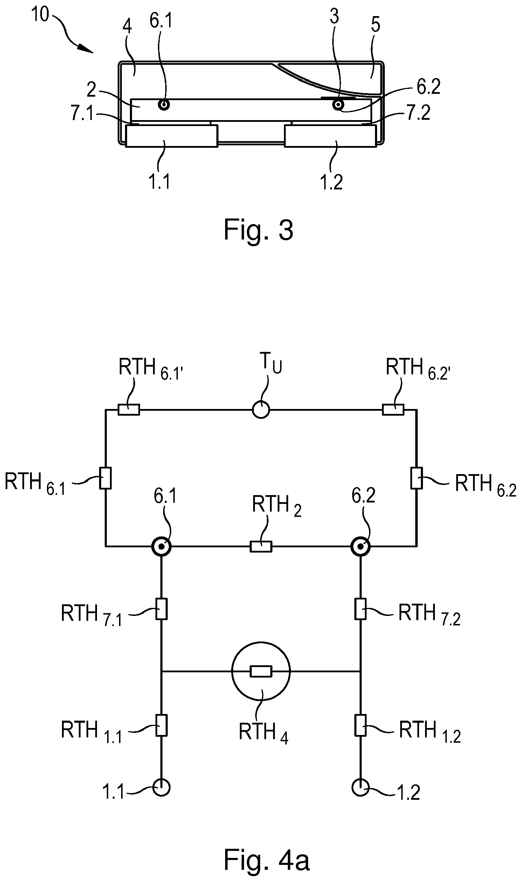

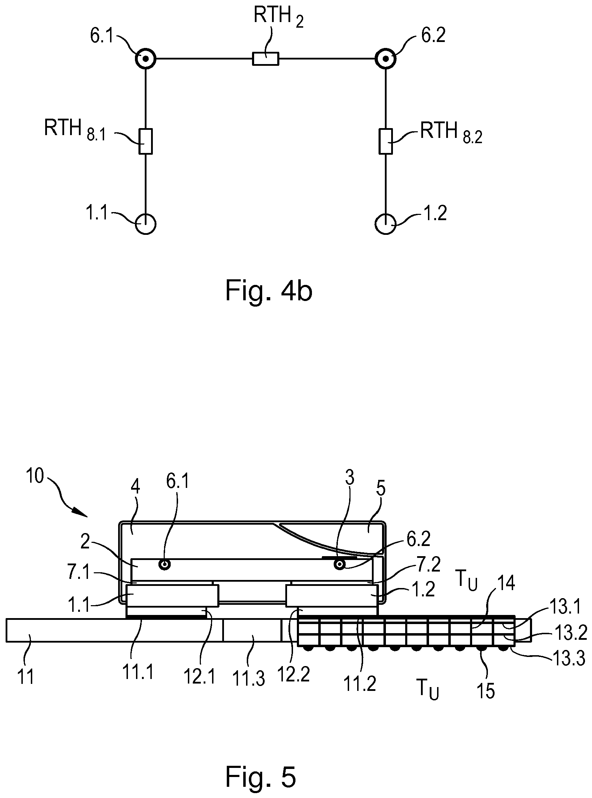

Ein erstes Ausführungsbeispiel der erfindungsgemäßen Sensor-Anordnung wird nachfolgend anhand der Figuren 1a, 1b, 2, 3 sowie 4a und 4b beschrieben. Dabei zeigen die Figuren 1a, 1b jeweils perspektivische Anordnungen derselbigen, Figur 2 eine Draufsicht auf die Anordnungsebene der Trägerelemente, Figur 3 eine seitliche Schnittansicht und die Figuren 4a, 4b thermische Ersatzschaltbilder der entsprechenden Sensor-Anordnung.

In der anschließenden Beschreibung von Ausführungsbeispielen anhand der erwähnten Figuren wird richtungsbeschreibende Terminologie wie z. B. "Oberseite", "Unterseite" etc. verwendet, um die räumliche Orientierung einzelner Komponenten der erfindungsgemäßen Sensoranordnung 10 zueinander zu charakterisieren. Da es grundsätzlich möglich ist, diese Komponenten im Rahmen der vorliegenden Erfindung auch anderweitig zueinander zu positionieren, ist die hierzu nachfolgend genutzte Terminologie in keinster Weise beschränkend zu verstehen.

Die erfindungsgemäße Sensor-Anordnung 10 weist einen Systemträger bzw. Leadframe auf, der im vorliegenden Ausführungsbeispiel zwei planare Trägerelemente 1.1, 1.2 bzw. Die-Pads umfasst, die aus elektrisch und thermisch leitfähigem Material bestehen. Vorliegend dient als Material für die Trägerelemente 1.1, 1.2 eine Kupfer-Legierung; alternativ hierzu wären beispielsweise auch Nickel-Eisen-Legierungen wie z.B. Alloy 42 an dieser Stelle einsetzbar. Ebenso könnten auch elektrisch nicht-leitfähige Materialien an dieser Stelle eingesetzt werden, die aber zumindest eine minimale thermische Leitfähigkeit besitzen sollten. Die beiden Trägerelemente 1.1, 1.2 sind in einer Anordnungsebene beabstandet voneinander angeordnet und weisen jeweils einen rechteckförmigen Querschnitt auf; als vorteilhaft erweist sich hier ein Mindestabstand von etwa 1mm. Auf den beiden Trägerelementen 1.1, 1.2 ist ein integrierter Signalverarbeitungsbaustein 2 bzw. ASIC angeordnet, wie dies insbesondere aus der seitlichen Schnittansicht in Figur 3 ersichtlich ist. Der plattenförmig ausgebildete Signalverarbeitungsbaustein 2 besitzt einen rechteckförmigen Querschnitt, wobei diejenigen Randbereiche des Signalverarbeitungsbausteins 2 auf den Trägerelementen 1.1, 1.2 aufliegen, die sich entlang der Rechtecks-Längsachse $L_2$ des Signalverarbeitungsbausteins 2 gegenüberliegen. Die Rechtecks-Längsachse $L_2$ des Signalverarbeitungsbausteins 2 ist hierbei, wie aus der Draufsicht auf die Anordnungsebene der Trägerelemente in Figur 2 ersichtlich, rechtwinklig zu den Längsachsen $L_{1.1}$, $L_{1.2}$ der beiden Trägerelemente 1.1, 1.2 orientiert. Wie ebenfalls in Figur 2 erkennbar, ist die Länge der Trägerelemente 1.1, 1.2 entlang ihrer jeweiligen Längsachsen $L_{1.1}$, $L_{1.2}$ jeweils etwas größer als die Breite des Signalverarbeitungsbausteins 2 senkrecht zu dessen Längsachse $L_2$. Die Trägerelemente 1.1, 1.2 ragen gemäß der Draufsicht-Schnittansicht der Figur 2 demzufolge entlang ihrer jeweiligen Längsachsen $L_{1.1}$, $L_{1.2}$ jeweils etwas über den Signalverarbeitungsbaustein 2 hinaus.

Zur Befestigung des Signalverarbeitungsbausteins 2 auf den Trägerelementen 1.1, 1.2 ist in den Auflagebereichen zwischen dem Signalverarbeitungsbaustein 2 und den Trägerelementen 1.1, 1.2 jeweils ein Befestigungsmaterial 7.1, 7.2 angeordnet, z.B. ein geeigneter Klebstoff bzw. ein geeignetes Die-Bond-Material. Als Befestigungsmaterial 7.1, 7.2 wird hierbei vorzugsweise ein Material mit einem möglichst geringen thermischen Widerstand gewählt.

Der Signalverarbeitungsbaustein 2 ist in der erfindungsgemäßen Sensor-Anordnung 10 somit nicht wie ansonsten üblich auf einem einzigen, durchgehenden Trägerelement angeordnet, sondern auf einem "gespaltenen" bzw. mehrteilig ausgebildeten Trägerelement, wobei im Ausführungsbeispiel zwei derartige Trägerelemente 1.1, 1.2 vorgesehen sind.

Ein gassensitiver Sensor 3 ist auf der Oberseite des Signalverarbeitungsbausteins 2 platziert und mit diesem elektrisch leitend verbunden. Der Sensor 3 ist im vorliegenden Ausführungsbeispiel als Feuchtesensor ausgebildet und liefert feuchteabhängige Signale, die vom Signalverarbeitungsbaustein 2 in weiterverarbeitbare Feuchtemesswerte umgewandelt werden. Als Feuchtesensor ist ein kapazitiver Feuchtesensor vorgesehen, der eine planare Grundelektrode sowie eine planare Deckelektrode umfasst und zwischen denen eine feuchteempfindliches Polymer angeordnet ist. Die darüber generierten Feuchtemesswerte können von einer nachgeordneten - nicht dargestellten - Folgeelektronik in verschiedenster Art und Weise weiterverarbeitet werden.

Der Signalverarbeitungsbaustein 2 und der gassensitive Sensor 3 werden in der erfindungsgemäßen Sensor-Anordnung 10 mindestens teilweise von einer Umkapselung 4 formschlüssig umschlossen. Die Formgebung der Umkapselung 4 erfolgt im Verlauf des Transfer-Molding-Herstellungsprozesses hierbei derart, dass eine Quaderform der Sensor-Anordnung 10 resultiert. Als Material für die Umkapselung 4 dient z.B. Epoxidharz. Grundsätzlich erweist sich als günstig, wenn das Umkapselungs-Material eine möglichst geringe Wärmeleitfähigkeit aufweist und somit als thermischer Isolator zwischen den beiden Trägerelementen 1.1, 1.2 fungiert. Im vorliegenden Ausführungsbeispiel mit vergleichbaren Dicken des Signalverarbeitungsbausteins 2 und der Trägerelemente 1.1, 1.2 wird ein Umkapselungs-Material mit einer Wärmeleitfähigkeit von ca. 1 W/(m • K) gewählt, die damit ca. 1/300 der Wärmeleitfähigkeit des i.w. aus Silizium bestehenden Signalverarbeitungsbausteins 2 beträgt.

Im dargestellten Ausführungsbeispiel der erfindungsgemäßen Sensor-Anordnung 10 besitzt der Quader einen quadratischen Querschnitt mit identischer Länge und Breite. In der Umkapselung 4 ist desweiteren eine Kavität 5 vorgesehen, über die für das zu messende Gas in der Umgebung der Sensor-Anordnung 10 ein Zugang zum Sensor 3 bereitgestellt wird. Die Kavität 5 erstreckt sich hierbei vom gassensitiven Sensor 3 bis zur oberen begrenzenden Seitenfläche der Umkapselung 4 und ist an einer Seite der quaderförmigen Umkapselung 4 geöffnet. Damit kann über diese seitliche Öffnung evtl. in der Kavität 5 befindliche Flüssigkeit ablaufen, so dass eine Verfälschung der Messung durch Flüssigkeit in der Kavität 5 verhindert werden kann.

Der Systemträger weist in diesem Ausführungsbeispiel desweiteren Anschlusskontakte 1.3 in Form sog. Leads auf, die nicht vollständig von der Umkapselung 4 umgeben und an zwei Seitenflächen der quaderförmigen Umkapselung 4 frei zugänglich sind. Über diese Anschlusskontakte 1.3 kann der Signalverarbeitungsbaustein 2 elektrisch kontaktiert und z.B. mit einer - nicht dargestellten - Folgeelektronik verbunden werden.

In den Signalverarbeitungsbaustein 2 sind in der erfindungsgemäßen Sensor-Anordnung 10 zwei Temperatursensoren 6.1, 6.2 integriert. Wie aus Figur 3 ersichtlich, sind die Temperatursensoren 6.1, 6.2 dabei in gegenüberliegenden Randbereichen des Signalverarbeitungsbausteins 2 platziert und damit jeweils benachbart zu einem der beiden Trägerelemente 1.1, 1.2 des Systemträgers angeordnet, nämlich oberhalb derselbigen.

Die Temperatursensoren 6.1, 6.2 sind jeweils als sog. bandgap-Temperatursensoren ausgebildet, in denen die Temperaturabhängigkeit der Vorwärtsspannung einer Diode, beispielsweise der pn-Übergang eines Transistors, ausgewertet wird. Wie aus den Figuren ersichtlich, sind die Temperatursensoren 6.1, 6.2 dabei an der Oberseite des Signalverarbeitungsbausteins 2 platziert. Die Art und Weise, wie die Temperatursensoren 6.1, 6.2 konkret ausgebildet werden, ist für die vorliegende Erfindung nicht wesentlich, d.h. es ist möglich die Temperatursensoren 6.1, 6.2 im Signalverarbeitungsbaustein 2 auch alternativ auszugestalten.

Über die beiden Temperatursensoren 6.1, 6.2 und deren Anordnung benachbart zu den Trägerelementen 1.1, 1.2 eines mehrteilig ausgebildeten Systemträgers ist es nunmehr möglich, je nach Anordnungs-Konfiguration z.B. auch Temperaturen in der Umgebung der erfindungsgemäßen Sensor-Anordnung 10 ohne zusätzliche Temperatursensoren messtechnisch zu erfassen; dies wird nachfolgend anhand eines konkreten Anwendungsbeispiels im Detail erläutert.

Darüber hinaus gibt es weitere Anwendungsmöglichkeiten für die erfindungsgemäße Sensor-Anordnung 10, entsprechende Möglichkeiten werden im Verlauf der folgenden Beschreibung ebenfalls noch skizziert. Es resultieren somit erweiterte Einsatzmöglichkeiten für die erfindungsgemäße Sensor-Anordnung.

Vor der Beschreibung eines derartigen Anwendungsbeispiels sei zum Verständnis desselbigen zunächst das thermische Ersatzschaltbild der vorhergehend beschriebenen Sensor-Anordnung anhand von Figur 4a erläutert. Dieses veranschaulicht angelehnt an die Geometrie des vorstehend beschriebenen Ausführungsbeispiels der erfindungsgemäßen Sensor-Anordnung 10 in genäherter Form dessen Wärmeübergangs-Verhalten. Um ein derartiges thermisches Gesamtmodell der Sensoranordnung zu erhalten, werden dabei bestimmte vernachlässigbare Wärmeleitungsbeiträge in der realen dreidimensionalen Struktur der Sensor-Anordnung nicht berücksichtigt.

Die noch nicht in den vorhergehenden Figuren erwähnten Größen in Figur 4a sind hierbei wie folgt definiert:

$RTH_{1.1}$ := Thermischer Widerstand des Trägerelements 1.1
$RTH_{1.2}$ := Thermischer Widerstand des Trägerelements 1.2
$RTH_{7.1}$ := Thermischer Widerstand des Befestigungsmaterials 7.1
$RTH_{7.2}$ := Thermischer Widerstand des Befestigungsmaterials 7.2
$RTH_4$ := Thermischer Widerstand des Umkapselungsmaterials zwischen Trägerelement 1.1 und Trägerelement 1.2
$RTH_2$ := Thermischer Widerstand des Signalverarbeitungsbausteins 2
$RTH_{6.1}$ := Thermischer Widerstand zwischen Temperatursensor 6.1 und Oberfläche des Signalverarbeitungsbausteins 2
$RTH_{6.2}$ := Thermischer Widerstand zwischen Temperatursensor 6.2 und Oberfläche des Signalverarbeitungsbausteins 2
$RTH_{6.1'}$ := Thermischer Übergangswiderstand zwischen Oberfläche des Signalverarbeitungsbausteins 2 und Umgebung im Bereich oberhalb des Temperatursensors 6.1
$RTH_{6.2'}$ := Thermischer Übergangswiderstand zwischen Oberfläche des Signalverarbeitungsbausteins 2 und Umgebung im Bereich oberhalb des Temperatursensors 6.2
$T_U$ := Temperatur in der Umgebung der Sensor-Anordnung 10

Aufgrund der Wahl eines Materials für die Umkapselung 4 mit einer sehr niedrigen Wärmeleitfähigkeit bzw. einem sehr hohen thermischen Widerstand $RTH_4$ resultiert zwischen den beiden Trägerelementen 1.1, 1.2 des Systemträgers in der Sensor-Anordnung 10 lediglich eine sehr geringe Wärmeleitung. Diese kann im praktischen Betrieb näherungsweise komplett vernachlässigt werden, da das gewählte Umkapselungsmaterial als thermischer Isolator zwischen den Trägerelementen 1.1, 1.2 wirkt. Da gleichzeitig die thermischen Übergangswiderstände $RTH_{6.1'}$ bzw. $RTH_{6.2'}$ zwischen der Oberfläche des Signalverarbeitungsbausteins 2 und der Umgebung im Bereich der Temperatursensoren 6.1, 6.2 sehr groß sind, ist es möglich, die Wärmeleitung zwischen den Temperatursensoren 6.1, 6.2 und der Umgebung über das Umkapselungsmaterial ebenfalls zu vernachlässigen.

Es ergibt sich für die anhand der Figuren 1a, 1b, 2 und 3 erläuterte Ausführungsform der erfindungsgemäßen Sensor-Anordnung 10 daher das vereinfachte thermische Ersatzschaltbild gemäß der Figur 4b. Die verbleibenden Größen im vereinfachten thermischen Ersatzschaltbild der erfindungsgemäßen Sensor-Anordnung 10 sind dabei wie folgt definiert:

$RTH_2$ := Thermischer Widerstand des Signalverarbeitungsbausteins 2 zwischen den Temperatursensoren 6.1, 6.2
$RTH_{8.1}$ := Thermischer Widerstand zwischen Temperatursensor 6.1 und Unterseite des Trägerelements 12.1
$RTH_{8.2}$ := Thermischer Widerstand zwischen Temperatursensor 6.2 und Unterseite des Trägerelements 12.2

Für die nachfolgende Erläuterung eines Anwendungsbeispiels der erfindungsgemäßen Sensor-Anordnung 10 wird das vereinfachte thermische Ersatzschaltbild gemäß Figur 4b verwendet.

Das in dieser Anwendung resultierende System ist in Figur 5 in einer seitlichen Schnittansicht schematisiert dargestellt. Die vorstehend erläuterte Sensor-Anordnung 10 ist hierbei auf einem plattenförmigen Substrat 11 angeordnet, welches als Leiterplatte aus FR4-Material ausgebildet ist. Dabei werden die auf der Unterseite der Sensor-Anordnung 10 freiliegenden Trägerelemente 1.1, 1.2 auf elektrisch leitfähigen Kontaktflächen 11.1, 11.2 des Substrats 11 platziert und dort befestigt. Die Befestigung erfolgt hierbei mittels eines Lötverfahrens und einem hierzu vorgesehenen Lotmaterial 12.1, 12.2. Alternativ wäre an dieser Stelle auch eine Klebe-Befestigung oder eine andere Befestigungsart einsetzbar, solange die entsprechende Verbindung einen möglichst geringen thermischen Widerstand aufweist.

Die Kontaktflächen 11.1, 11.2 auf dem Substrat 11 bestehen aus dünnen Kupferschichten, die jeweils räumlich begrenzt auf der Oberseite des Substrats 11 angeordnet sind. Zwischen den Kontaktflächen 11.1, 11.2 des ersten und zweiten Trägerelements 1.1, 1.2 ist im Substrat 11 eine Öffnung 11.3 vorgesehen, die vorliegend kreisförmig ausgebildet ist.

Im Bereich unterhalb des ersten Trägerelements 1.1 bzw. ersten Temperatursensors 6.1 weist die Kontaktfläche 11.1 dabei eine geometrische Form auf, die derjenigen des ersten Trägerelements 1.1 entspricht und ist demzufolge rechteckförmig ausgebildet.

Unterschiedlich hierzu ist demgegenüber der Bereich unterhalb des zweiten Trägerelements 1.2 bzw. des zweiten Temperatursensors 6.2 ausgebildet. So sind hier im bzw. auf dem Substrat 11 im Bereich benachbart zum zweiten Trägerelement 1.2 verschiedenste Wärmeleitungsmittel angeordnet, über die ein verbesserter Wärmeübergang zwischen dem zweiten Temperatursensor 6.2 und der unmittelbaren Substratumgebung in diesem Bereich sichergestellt werden kann. Angestrebt wird durch die entsprechenden Wärmeleitungsmittel demzufolge ein möglichst geringer thermischer Durchgangswiderstand bzw. Wärmedurchgangswiderstand zwischen dem zweiten Temperatursensor 6.2 und der unmittelbaren Substratumgebung. Als thermischer Durchgangswiderstand bzw. Wärmedurchgangswiderstand sei hierbei die Summe der resultierenden thermischen Widerstände und der thermischen Übergangswiderstände entlang der entsprechenden Strecke verstanden.

Zu den Wärmeleitungsmitteln gehören vorliegend mehrere parallel zueinander angeordnete Metallschichten 11.2, 13.1 - 13.3 auf und im Substrat 11. Neben der auf der Oberseite des Substrats 11 angeordneten Metallschicht 11.2, die gleichzeitig die Kontaktfläche für das zweite Trägerelement 1.2 bildet, sind im dargestellten Ausführungsbeispiel zwei weitere Metallschichten 13.1, 13.2 im Inneren des Substrats 11 sowie eine Metallschicht 13.3 auf der Unterseite des Substrats 11 vorgesehen. Die Metallschichten 11.2, 13.1 - 13.3 erstrecken sich jeweils über einen Bereich im Substrat 11, der deutlich größer als die Auflagefläche der Sensor-Anordnung 10 bzw. die Kontaktfläche 11.2 des zweiten Trägerelements 1.2 ist.

Desweiteren sind als Wärmeleitungsmittel zur Optimierung der Wärmeleitung im vorliegenden Ausführungsbeispiel Durchkontaktierungen 14 vorgesehen, die als senkrecht zur Substrat-Oberfläche orientierte Bohrungen mit kreisförmigem Querschnitt im Bereich der Metallschichten 11.2, 13.1 - 13.3 das Substrat 11 durchsetzen und zumindest teilweise mit einem Material mit hoher Wärmeleitfähigkeit verfüllt sind; vorliegend ist als Material zum Verfüllen der Durchkontaktierungen 14 Kupfer vorgesehen, das etwa mindestens auf der Innenseite der Bohrungen angeordnet ist. Darüber hinaus ist es natürlich möglich, die Bohrungen komplett mit Kupfer oder einem anderen geeigneten Material zu verfüllen. Über die Durchkontaktierungen 14 wird auf diese Art und Weise ein guter Wärmeübergang zwischen der Ober- und Unterseite des Substrats 11 sichergestellt.

Ferner umfassen im vorliegenden Ausführungsbeispiel die erwähnten Wärmeleitungsmittel noch mehrere metallische Lötpunkte 15, die auf der Ober- und Unterseite des Substrats 11 jeweils im Bereich der nichtbedeckten Metallschichten 11.1, 13.3 angeordnet sind.

Neben den in diesem Ausführungsbeispiel vorgesehenen Wärmeleitungsmitteln wäre es desweiteren möglich, auf dem Substrat 11 einen SMD-bestückbaren Kühlkörper als Wärmeleitungsmittel anzuordnen, um darüber noch zusätzlich den thermischen Durchgangswiderstand zwischen dem zweiten Temperatursensor 6.2 und der zur Umgebung des Systems zu minimieren.

Hinsichtlich der Wärmeleitungsmittel stehen demnach verschiedenste Möglichkeiten zur Verfügung, die je nach Anwendungsfall flexibel eingesetzt werden können.

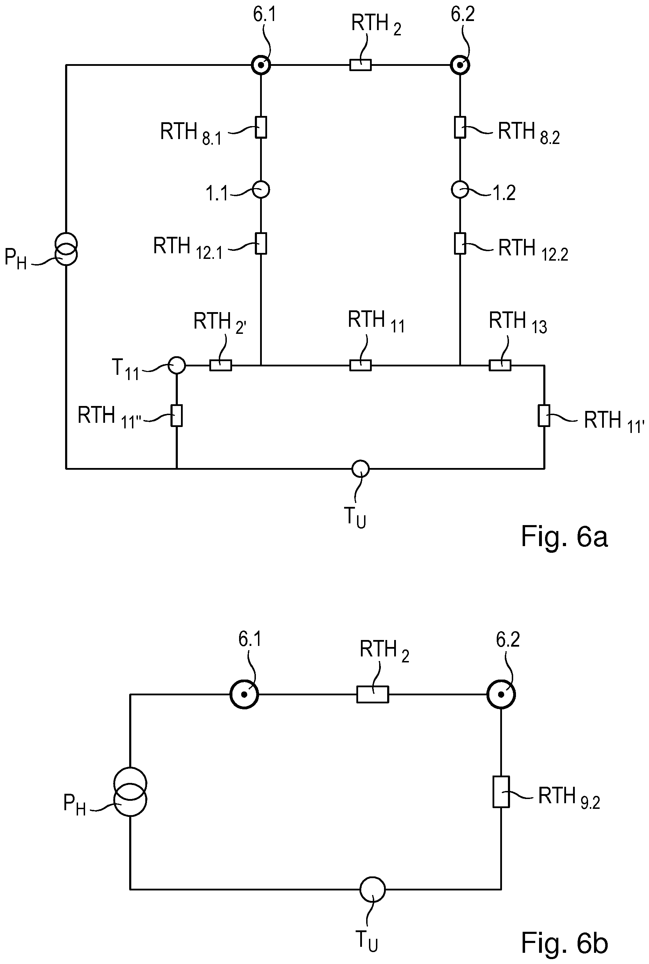

Das thermische Ersatzschaltbild des Systems aus Figur 5 ist in Figur 6a dargestellt, wobei die verschiedenen Größen in Figur 6a wie folgt definiert sind:

$RTH_2$ := Thermischer Widerstand des Signalverarbeitungsbausteins 2

$P_H$ := Heizleistung des Signalverarbeitungsbausteins 2

$RTH_{8.1}$ := Thermischer Widerstand zwischen Signalverarbeitungsbaustein 2 und unterer Kontaktierungsfläche des Trägerelements 1.1

$RTH_{8.2}$ := Thermischer Widerstand zwischen Signalverarbeitungsbaustein 2 und unterer Kontaktierungsfläche des Trägerelements 1.2

$RTH_{12.1}$ := Thermischer Widerstand zwischen unterer Kontaktierungsfläche des Trägerelements 1.1 und Kontaktbereich 11.1 auf Substrat 11

$RTH_{2.2}$ := Thermischer Widerstand zwischen unterer Kontaktierungsfläche des Trägerelements 1.2 und Kontaktbereich 11.2 auf Substrat 11

$RTH_{11}$ := Thermischer Widerstand des Substrats 11 zwischen den Kontaktbereichen 11.1, 11.2 der Trägerelemente 1.1, 1.2

$RTH_{2'}$ := Thermischer Widerstand zwischen Trägerelement 1.1 und Stelle, an der das Substrat 11 Wärme an die Umgebung abgibt

$RTH_{13}$ := Thermischer Widerstand der Metallschichten 11.2, 13.1 - 13.3 und Durchkontaktierungen 14

$RTH_{11'}$ := Thermischer Übergangswiderstand zwischen Wärmeleitungsmitteln im Substrat 11 und Umgebung im Bereich benachbart zur zweiten Kontaktfläche 11.2

$RTH_{11''}$ := Thermischer Übergangswiderstand zwischen Substrat 11 und Umgebung im Bereich benachbart zur ersten Kontaktfläche 11.1

$T_U$ := Temperatur in der Umgebung der Nähe der Sensor-Anordnung im Bereich benachbart zur zweiten Kontakt-

fläche 11.2

$T_{11}$ := Temperatur des Substrats 1

Betrachtet man in der Praxis die Werte für die verschiedenen thermischen Widerstände aus dem Ersatzschaltbild der Figur 6a, so stellt sich heraus, dass die Wärmeleitung im Substrat 11 praktisch vernachlässigbar ist. Damit lässt sich wiederum ein vereinfachtes thermisches Ersatzschaltbild für das System aus Figur 5 erstellen; dieses ist in Figur 6b dargestellt. Die verbleibenden Größen im vereinfachten thermischen Ersatzschaltbild dieses Systems sind dabei wie folgt definiert:

$RTH_2$ := Thermischer Widerstand des Signalverarbeitungsbausteins 2

$P_H$ := Heizleistung des Signalverarbeitungsbausteins

$RTH_{9.2}$ := Wärmedurchgangswiderstand zwischen Temperatursensor 6.2 und Umgebung

$T_U$ := Temperatur in der Umgebung der Sensor-Anordnung 10

Aus dem thermischen Ersatzschaltbild in Figur 6b ist ersichtlich, dass der sich ausbildende Temperaturunterschied zwischen den beiden Temperatursensoren 6.1, 6.2 unabhängig von der Strömungsgeschwindigkeit einer Luftströmung ist, die das System aus Figur 5 umströmt. Auch wenn der Wärmedurchgangswiderstand $RTH_{9.2}$ von der Geschwindigkeit der entsprechenden Luftströmung abhängig ist, bleibt der sich über dem thermischer Widerstand $RTH_2$ des Signalverarbeitungsbausteins 2 sich ausbildende Temperaturunterschied gleich. Dieser Temperaturunterschied ist nur von der durch den Signalverarbeitungsbaustein 2 erzeugten Heizleistung abhängig, die dort etwa durch die Signalverarbeitung im Digitalteil resultiert. Es lässt sich daher aus der Messung des Temperaturunterschieds zwischen den beiden Temperatursensoren 6.1, 6.2 im Signalverarbeitungsbaustein 2 bei bekanntem Wärmedurchgangswiderstand $RTH_{9.2}$ (ruhende Luft) die Umgebungstemperatur $T_U$ in der unmittelbaren Umgebung des Systems aus Figur 5 bestimmen, wie nachfolgend beispielhaft erläutert sei.

So seien für ein derartiges System der thermische Widerstand $RTH_2$ im Signalverarbeitungsbaustein 2 und der Wärmedurchgangswiderstand $RTH_{9.2}$ zwischen dem Temperatursensor 6.2 und der Umgebung folgendermaßen gegeben:

$RTH_2$ = 35 K/W

$RTH_{9.2}$ = 140 K/W (ruhende Luft)

An den beiden Temperatursensoren 6.1, 6.2 werden im Betrieb die nachfolgenden Temperaturen $T_1$, $T_2$ gemessen:

$T_1$ = 27°C

$T_2$ = 26°C

Aufgrund der Temperaturdifferenz $\Delta T = T_1 - T_2$ resultiert nach dem Ohmschen Gesetz der Wärmeleitung folgender Wärmestrom $\Phi$ zwischen den beiden Temperatursensoren 6.1, 6.2:

$$\Phi = \Delta T / RTH_2 = 1 \text{ K} / 35 \text{ K/W} = 0{,}028571 \text{ W}$$

Der identische Wärmestrom $\Phi$ resultiert auch zwischen dem Temperatursensor 6.2 und der Umgebung der Sensor-Anordnung 10, d.h. es gilt:

$$\Phi = (T_2 - T_U) / RTH_{9.2}$$

Daraus lässt sich dann schließlich die Umgebungstemperatur $T_U$ folgendermaßen bestimmen:

$$T_U = T_2 - \Phi \cdot RTH_{9.2} = 26°C - 0{,}028571 \text{ W} \cdot 140 \text{ K/W} = 22°C$$

Die erfindungsgemäß ausgebildete Sensor-Anordnung 10 ermöglicht somit in diesem Anwendungsbeispiel die Bestimmung der Umgebungstemperatur $T_U$ benachbart zur Sensor-Anordnung 30 aus der Temperaturmessung über die beiden im Signalverarbeitungsbaustein 2 integrierten Temperatursensoren 6.1, 6.2; ein separater bzw. zusätzlicher Temperatursensor ist hierzu nicht erforderlich.

Wäre gegenüber dem erläuterten Beispiel der Wärmedurchgangswiderstand $RTH_{9.2}$ nicht konstant und damit auch nicht bekannt, etwa wegen einer vorhandenen Luftströmung, so kann durch Einbringen einer definierten Heizleistung auf den jeweiligen Wärmedurchgangswiderstand $RTH_{9.2}$ zurückgerechnet und die Umgebungstemperatur $T_U$ bestimmt werden.

Neben diesem Anwendungsbeispiel der erfindungsgemäßen Sensor-Anordnung existieren im Rahmen der vorliegenden Erfindung selbstverständlich noch weitere vorteilhafte Anwendungsmöglichkeiten.

Auch im Hinblick auf die konkrete Ausbildung der erfindungsgemäßen Sensor-Anordnung gibt es neben dem erläuterten Ausführungsbeispiel noch weitere Realisierungsmöglichkeiten.

So ist es etwa auch möglich, mehr als zwei Trägerelemente in der Sensor-Anordnung vorzusehen.

Analog hierzu können natürlich auch mehr als zwei Temperatursensoren im Signalverarbeitungsbaustein integriert sein usw..

**Patentansprüche**

1. Sensor-Anordnung mit

   - einem Systemträger, der mindestens ein erstes planares Trägerelement (1.1) und ein zweites planares Trägerelement (1.2) umfasst, wobei die Trägerelemente (1.1, 1.2) in einer Anordnungsebene beabstandet voneinander angeordnet sind,
   - einem integrierten Signalverarbeitungsbaustein (2), der auf den Trägerelementen (1.1, 1.2) angeordnet ist,
   - einer Umkapselung (4), die den Signalverarbeitungsbaustein (2) mindestens teilweise umschließt und
   - mindestens zwei im Signalverarbeitungsbaustein (2) integrierten Temperatursensoren (6.1, 6.2), wobei je ein Temperatursensor (6.1, 6.2) benachbart zu einem Trägerelement (1.1, 1.2) angeordnet ist.

2. Sensor-Anordnung nach Anspruch 1, wobei gegenüberliegende Randbereiche des Signalverarbeitungsbausteins (2) auf den Trägerelementen (1.1, 1.2) aufliegen.

3. Sensor-Anordnung nach Anspruch 2, wobei in den Auflagebereichen zwischen dem Signalverarbeitungsbaustein (2) und den Trägerelementen (1.1, 1.2) ein Befestigungsmaterial angeordnet ist, welches einen geringen thermischen Widerstand besitzt.

4. Sensor-Anordnung nach Anspruch 2, wobei der Signalverarbeitungsbaustein (2) einen rechteckförmigen Querschnitt aufweist und diejenigen Randbereiche des Signalverarbeitungsbausteins (2) auf den Trägerelementen (1.1, 1.2) aufliegen, die sich entlang der Rechtecks-Längsachse gegenüberliegen.

5. Sensor-Anordnung nach Anspruch 4, wobei die Trägerelemente (1.1, 1.2) jeweils einen rechteckförmigen Querschnitt aufweisen und die Längsachsen der Trägerelemente (1.1, 1.2) rechtwinklig zur Längsachse des Signalverarbeitungsbausteins (2) angeordnet sind.

6. Sensor-Anordnung nach Anspruch 5, wobei die Länge der Trägerelemente (1.1, 1.2) entlang ihrer Längsachse jeweils größer als die Breite des Signalverarbeitungsbausteins (2) senkrecht zur Rechtecks-Längsachse ist.

7. Sensor-Anordnung nach mindestens einem der vorhergehenden Ansprüche, wobei die Trägerelemente (1.1, 1.2) in der Anordnungsebene einen gegenseitigen Abstand zueinander aufweisen, der derart gewählt ist, dass eine thermische Entkopplung zwischen den Trägerelementen (1.1, 1.2) gewährleistet ist.

8. Sensor-Anordnung nach Anspruch 7, wobei zwischen den Trägerelementen (1.1, 1.2) ein Material angeordnet ist, das eine geringe Wärmeleitfähigkeit aufweist.

9. Sensor-Anordnung nach mindestens einem der vorhergehenden Ansprüche, wobei die Trägerelemente (1.1, 1.2) aus einer Kupfer-Legierung oder einer Nickel-Eisen-Legierung bestehen.

10. Sensor-Anordnung nach Anspruch 1, wobei der Bereich zwischen den Trägerelementen (1.1, 1.2) baulich derart ausgebildet ist, dass dort eine verringerte Wärmeleitfähigkeit gegenüber den Trägerelementen (1.1, 1.2) resultiert.

11. Sensor-Anordnung nach mindestens einem der vorhergehenden Ansprüche, wobei auf dem Signalverarbeitungsbaustein (2) ein gassensitiver Sensor (3) angeordnet und mit diesem elektrisch leitfähig verbunden ist.

12. Sensor-Anordnung Anspruch 11, wobei die Umkapselung (4) quaderförmig ausgebildet ist und den Systemträger mit den Trägerelementen (1.1, 1.2) und dem Signalverarbeitungsbaustein (2) mit Ausnahme einer Kavität (5) formschlüssig umgibt und die Kavität (5) sich vom Sensor (3) bis zu einer begrenzenden Seitenfläche der Umkapselung (4) erstreckt.

13. System, bestehend aus einer Sensor-Anordnung nach mindestens einem der vorhergehenden Ansprüche, angeordnet auf einem plattenförmigen Substrat (11), wobei das Substrat (11) im Bereich eines Trägerelements (1.2) Wärmeleitungsmittel aufweist, über die ein verbesserter Wärmeübergang zwischen dem Substrat (11) und der Umgebung erzielbar ist.

14. System nach Anspruch 13, wobei die Wärmeleitungsmittel mindestens eine zusätzliche Metallschicht (13.1 - 13.3) im Substrat (11) umfassen.

15. System nach Anspruch 13, wobei die Wärmeleitungsmittel mehrere Durchkontaktierungen (14) im Substrat (11) umfassen, die zumindest teilweise mit einem Material hoher Wärmeleitfähigkeit gefüllt sind.


**Claims**

1. Sensor arrangement having

   - a system carrier which comprises at least one first planar carrier element (1.1) and one second planar carrier element (1.2), wherein the carrier elements (1.1, 1.2) are arranged at a distance from one another in an arrangement plane,
   - an integrated signal-processing module (2) which is arranged on the carrier elements (1.1, 1.2),
   - an encapsulation (4) which at least partially surrounds the signal-processing module (2), and
   - at least two temperature sensors (6.1, 6.2) which are integrated in the signal-processing module (2), wherein in each case one temperature sensor (6.1, 6.2) is arranged adjacent to one carrier element (1.1, 1.2).

2. Sensor arrangement according to Claim 1, wherein opposite edge regions of the signal-processing module (2) rest on the carrier elements (1.1, 1.2).

3. Sensor arrangement according to Claim 2, wherein a fastening material, which has a low thermal resistance, is arranged in the support regions between the signal-processing module (2) and the carrier elements (1.1, 1.2).

4. Sensor arrangement according to Claim 2, wherein the signal-processing module (2) has a rectangular cross section and those edge regions of the signal-processing module (2) which are situated opposite one another along the longitudinal axis of the rectangle rest on the carrier elements (1.1, 1.2).

5. Sensor arrangement according to Claim 4, wherein the carrier elements (1.1, 1.2) each have a rectangular cross section, and the longitudinal axes of the carrier elements (1.1, 1.2) are arranged at a right angle in relation to the longitudinal axis of the signal-processing module (2).

6. Sensor arrangement according to Claim 5, wherein the length of the carrier elements (1.1, 1.2) along their longitudinal axis is greater than the width of the signal-processing module (2) perpendicular to the longitudinal axis of the rectangle in each case.

7. Sensor arrangement according to at least one of the preceding claims, wherein the carrier elements (1.1, 1.2) are at a mutual distance from one another in the arrangement plane, the said distance being selected in such a way that thermal decoupling between the carrier elements (1.1, 1.2) is ensured.

8. Sensor arrangement according to Claim 7, wherein a material which has a low thermal conductivity is arranged between the carrier elements (1.1, 1.2).

9. Sensor arrangement according to at least one of the preceding claims, wherein the carrier elements (1.1, 1.2) are composed of a copper alloy or a nickel/iron alloy.

10. Sensor arrangement according to Claim 1, wherein the region between the carrier elements (1.1, 1.2) is structurally designed in such a way that a reduced thermal conductivity in relation to the carrier elements (1.1, 1.2) results there.

11. Sensor arrangement according to at least one of the preceding claims, wherein a gas-sensitive sensor (3) is arranged on the signal-processing module (2) and is electrically conductively connected to the said signal-processing module.

12. Sensor arrangement Claim 11, wherein the encapsulation (4) is of cuboidal design and surrounds the system carrier comprising the carrier elements (1.1, 1.2) and the signal-processing module (2), with the exception of a cavity (5), in an interlocking manner, and the cavity (5) extends from the sensor (3) as far as a delimiting side face of the encapsulation (4).

13. System, comprising a sensor arrangement according to at least one of the preceding claims, arranged on a plate-like substrate (11), wherein the substrate (11) has heat-conducting means in the region of a carrier element (1.2), it being possible for improved heat transfer between the substrate (11) and the surrounding area to be achieved by means of the said heat-conducting means.

14. System according to Claim 13, wherein the heat-conducting means comprise at least one additional metal layer (13.1-13.3) in the substrate (11).

15. System according to Claim 13, wherein the heat-conducting means comprise a plurality of plated through-holes (14) in the substrate (11), the said plated through-holes being at least partially filled with a material of high thermal conductivity.

**Revendications**

1. Système de capteur comportant

   - un support de système qui comprend au moins un premier élément de support plan (1.1) et un second élément de support plan (1.2), dans lequel les éléments de support (1.1, 1.2) sont disposés de manière espacée les uns des autres dans un plan d'agencement,
   - un composant de traitement de signaux (2) intégré, qui est disposé sur les éléments de support (1.1, 1.2),
   - une encapsulation (4) qui entoure le composant de traitement de signaux (2) au moins partiellement, et
   - au moins deux capteurs de température (6.1, 6.2) intégrés dans le composant de traitement de signaux (2), dans lequel un capteur de température (6.1, 6.2) est respectivement prévu au voisinage d'un élément de support (1.1, 1.2).

2. Système de capteur selon la revendication 1, dans lequel des zones de bord opposées du composant de traitement de signaux (2) reposent sur les éléments de support (1.1, 1.2).

3. Système de capteur selon la revendication 2, dans lequel un matériau de fixation qui possède une faible résistance thermique est prévu dans les zones d'appui entre le composant de traitement de signaux (2) et les éléments de support (1.1, 1.2).

4. Système de capteur selon la revendication 2, dans lequel le composant de traitement de signaux (2) présente une section transversale rectangulaire et dans lequel les zones de bord du composant de traitement de signaux (2) reposent sur les éléments de support (1.1, 1.2) qui sont opposés les uns aux autres le long de l'axe longitudinal du rectangle.

5. Système de capteur selon la revendication 4, dans lequel les éléments de support (1.1, 1.2) présentent respectivement une section transversale rectangulaire et dans lequel les axes longitudinaux des éléments de support (1.1, 1.2) sont disposés à angle droit par rapport à l'axe longitudinal du composant de traitement de signaux (2).

6. Système de capteur selon la revendication 5, dans lequel la longueur des éléments de support (1.1, 1.2) suivant leur axe longitudinal est respectivement supérieure à la largeur du composant de traitement de signal (2) perpendiculairement à l'axe longitudinal du rectangle.

7. Système de capteur selon au moins l'une des revendications précédentes, dans lequel les éléments de support (1.1, 1.2) présentent dans le plan d'agencement une distance mutuelle qui est sélectionnée de manière à assurer un découplage thermique entre les éléments de support (1.1, 1.2).

8. Système de capteur selon la revendication 7, dans lequel il est prévu entre les éléments de support (1.1, 1.2) un matériau qui présente une faible conductivité thermique.

9. Système de capteur selon au moins l'une des revendications précédentes, dans lequel les éléments de support (1.1, 1.2) sont constitués d'un alliage de cuivre ou d'un alliage de nickel-fer.

10. Système de capteur selon la revendication 1, dans lequel la zone comprise entre les éléments de support (1.1, 1.2) est réalisée structurellement de manière à ce qu'il en résulte à cet endroit une conductivité thermique réduite par rapport aux éléments de support (1.1, 1.2).

11. Système de capteur selon au moins l'une des revendications précédentes, dans lequel il est prévu sur le composant de traitement de signaux (2) un capteur sensible au gaz (3) et qui est relié à celui-ci de manière électriquement conductrice.

12. Système de capteur la revendication 11, dans lequel l'encapsulation (4) est réalisée sous une forme parallélépipédique et entoure par complémentarité de forme le support de système comportant les éléments de support (1.1, 1.2) et le composant de traitement de signaux (2) à l'exclusion d'une cavité (5) et la cavité (5) s'étend depuis le capteur (3) jusqu'à une surface latérale de délimitation de l'encapsulation (4).

13. Système constitué d'un système de capteur selon au moins l'une des revendications précédentes, disposé sur un substrat en forme de plaque (11), dans lequel le substrat (11) présente, dans la zone d'un élément de support (1.2), des moyens thermiquement conducteurs par l'intermédiaire desquels une transmission thermique améliorée peut être obtenue entre le substrat (11) et l'environnement.

14. Système selon la revendication 13, dans lequel les moyens thermiquement conducteurs comprennent au moins une couche métallique supplémentaire (13.1-13.3) dans le substrat (11).

15. Système selon la revendication 13, dans lequel les moyens thermiquement conducteurs comprennent plusieurs contacts traversants (14) dans le substrat (11) qui sont remplis au moins partiellement d'un matériau ayant une conductivité thermique plus élevée.

Fig. 1a

Fig. 1b

Fig. 2

Fig. 3

Fig. 4a

Fig. 4b

Fig. 5

Fig. 6a

Fig. 6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2657691 A1

- DE 202014102842 U1